# EUROPEAN PATENT APPLICATION

(11) **EP 4 450 638 A1**
(43) Date of publication of application: **23.10.2024**
(21) Application number: 23168397.0
(22) Date of filing: 18.04.2023
(51) Int. Cl.: C12Q 1/6825, G01N 33/52

(54) **LABEL AND MARKER STRUCTURE FOR MARKING A TARGET STRUCTURE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A label (100, 102, 206, 208, 514) for marking a target structure (508, 510) is provided, comprising at least one polyyne (104, 110) and at least one oligonucleotide (106), wherein the at least one polyyne (104, 110) is configured to be detectable via a distinct Raman spectroscopy characteristic. In a further aspect, a method for examining a target structure (508, 510) marked with a marker structure (200, 300, 400, 500, 502) comprising the label is provided.

## Description

### Technical field

The invention relates to a label for marking a target structure, a marker structure comprising a plurality of the labels and a method for examining a target structure marked with the marker structure.

### Background

In order to address key problems in the field of life sciences it is vital to precisely detect the presence of a target structure, such as a molecule or a protein, in a biological sample (e.g. tissue samples or cell cultures), an environmental sample (a soil sample), a water sample, a diagnostic procedure (e.g. in a solid or liquid biopsy or a sample prepared from either) or a lysate or extract from such a sample. This can be done by introducing marker structures into the sample that bind to specific target structures, e.g. specific biomolecules. These markers typically comprise an affinity reagent that attaches to the structure in question and a fluorescent dye that is either directly conjugated to the affinity reagent or attached to the affinity reagent by means of a secondary affinity reagent. There are various techniques for analysing biological samples prepared in this way. The plexing level in fluorescence microscopy, i.e. the number of different fluorescent dyes that can be read out at the same time is generally low and in the case of fluorescence microscopy typically in the range of 1-5 dyes for channel-based readouts and 5-12 dyes for readouts with spectral detectors, which use dispersive optical elements, such as prisms or gratings in combination with multiple detectors or array detectors. In fluorescence-based cytometry and sorting, somewhat higher plexing levels have been attained, but also in this case plexing is limited to a low number of dyes and consequently markers that can be readout in one experiment.

Multiplexing in microscopy, cytometry and other application areas is limited by the spectral overlap of fluorescence emission and excitation. It is therefore desirable to further increase the number of parameters that may be varied to allow for higher plexing levels.

### Summary

It is an object to provide a label that is distinguishable from fluorescent labels and allows the simple generation of compact marker structures.

The afore-mentioned object is achieved by a label according to claim 1. Advantageous embodiments are defined in the dependent claims and the following description.

Specifically, a label for marking a target structure is provided, comprising at least one polyyne and at least one oligonucleotide, wherein the at least one polyyne is configured to be detectable via a distinct Raman spectroscopy characteristic.

Thus, the label is detectable by Raman spectroscopy and distinguishable from typically used fluorescent labels. Polyynes are small molecules that result in a compact label that is easily and flexibly attached by means of the oligonucleotide.

Preferably, the distinct Raman spectroscopy characteristic is a stimulated Raman scattering (SRS) characteristic or a coherent anti-Stokes Raman scattering (CARS) characteristic. For example, the Raman spectroscopy characteristic may be measured by means of a Raman microscope.

Preferably, the label further comprises at least one first fluorophore which is configured to be detectable via at least a distinct spectral characteristic. This enables generating labels that are distinguishable by a larger variety of parameters. The spectral characteristic of the first fluorophore may for example be an excitation wavelength and/or an emission wavelength.

Preferably, the label further comprises at least one second fluorophore which is configured to be detectable via at least a distinct fluorescence lifetime characteristic. This enables generating labels that are distinguishable by a larger variety of parameters. Additionally, the first and second fluorophore may be detectable by a respective other distinct fluorescent characteristic. For example, the first fluorophore may be distinguishable by a distinct fluorescence lifetime characteristic.

Preferably, the label comprises at least one conjugate which is formed from the at least one polyyne and the at least one oligonucleotide.

Preferably, the at least one conjugate further includes the at least one first fluorophore and/or the at least one second fluorophore.

Preferably, the label is configured to be attached to an affinity reagent binding to the target structure. This enables specific binding of the label to the target structure. For example, the oligonucleotide of the label may attach to a complementary sequence of the affinity reagent. The affinity reagent may be an antibody with specific affinity to the target structure.

According to another aspect, a marker structure is provided comprising a plurality of the labels. The plurality of labels enables generating a more diverse set of individually distinguishable marker structures.

Preferably, the labels of the plurality of labels differ from each other at least in part with respect to the distinct Raman spectroscopy characteristics of their respective polyynes, and/or the spectral characteristics of their first fluorophores, and/or the fluorescence lifetime characteristics of their second fluorophores. This enables generating a larger number of distinguishable marker structures.

Preferably, the marker structure comprises labels only comprising fluorophores. This enables generating a larger number of distinguishable marker structures.

Preferably, the marker structure comprises a backbone on which the labels are attached. This enables a particularly robust marker structure.

Preferably, the backbone is formed from a DNA nanotechnology-based structure or the backbone is formed from a gel-like structure. This enables a particular flexible assembly of the marker structure. The gel-like structure may in particular comprise polyethylene glycol (PEG).

For example, the backbone comprises scaffold strands, and staple strands configured to bind to the scaffold strands at predetermined positions to fold the scaffold strand into a predetermined shape. Such a backbone may be a DNA-origami. These DNA origami structures may range in size from a few nanometres into the micron range. For the fabrication of such DNA origami-based structures longer DNA molecules (scaffold strands) are folded at precisely identified positions by so called staple strands. The DNA origami may be designed to provide a self-assembly backbone of a particular predetermined shape. This enables an easy and reproducible synthesis and assembly of the backbone. Staple strands may be position-selectively functionalised. The positional resolution in this case is limited by the size of a nucleotide, which is in the range of a nanometre or below. This has been exploited in the prior art to generate fluorescent standards, wherein fluorescent dyes are connected to precisely located bands on the DNA origami. These standards are known as "nanoruler" and are used for the calibration of imaging systems like confocal or super resolution microscopes (e.g. STED), for example, as disclosed by US2014/0057805 A1.

The DNA origami provides a scaffold for the labels. Preferably, the DNA origami structure comprises at least one scaffold strand and multiple staple strands, wherein the staple strands are complementary to at least parts of the scaffold strand and configured to bring the scaffold strand into a predetermined conformation. In particular, the strands are oligonucleotides. This enables generating backbones with predetermined two- or three-dimensional shapes that can self-assemble. Further, this enables the site-specific placement of attachment sites for labels on the backbone.

The attachment sites being unique nucleic acid sequences, preferably of the staple strands. Preferably, the labels may be attached to staple strands of the backbone at predetermined attachment sites. Since the staple strands are located at predetermined positions the positions of the attachment sites may equally be predetermined. Thus, the attachment site (of the nucleic acid backbone) is a unique oligonucleotide sequence complementary to the attachment oligonucleotide portion of one label.

Alternatively, the backbone may comprise or consist of DNA bricks. These DNA bricks are shorter length oligonucleotides that have overlapping hybridising stretches in order to bind to each other and assemble the backbone. In this case, the oligonucleotides of the labels may form an integral part of the nucleic acid backbone. Alternatively, attachment sites on the shorter length oligonucleotides of the DNA bricks may allow binding of the oligonucleotides of the labels.

Preferably, the first fluorophores and/or the second fluorophores, of the respective labels, are excitonically decoupled by an interleaved arrangement with the polyynes along or around the backbone. This enables particularly precise measurement of the fluorescent properties of the fluorophores of the labels attached to the marker structure.

In a further aspect, a method for examining a target structure is provided, comprising the following steps: marking the target structure with the marker structure; measuring Raman spectroscopy characteristic of the marked target structure; and measuring fluorescence of the marked target structure.

The measured Raman spectroscopy characteristic is, for example, a stimulated Raman scattering (SRS) characteristic or a coherent anti-Stokes Raman scattering (CARS) characteristic.

Preferably, measuring fluorescence of the marked target structure comprises detecting spectral characteristics of the marker structure and/or detecting fluorescence lifetime characteristics of the marker structure. This enables distinguishing between a larger number of marker structures.

Preferably, at least one of the Raman spectroscopy characteristic and the fluorescence are measured, or imaged, by means of a microscope. In particular, this enables generating respective images of the target structure and localising the marked target structure.

Preferably, at least one of the Raman spectroscopy characteristic and the fluorescence are measured by means of a cytometer. This enables identifying marked target structures particularly quickly.

Preferably, the method further comprises the step of sorting the marked target structure based on the measured Raman spectroscopy characteristic and/or the measured fluorescence. For example, fluorescence-activated cell sorting (FACS) based on measuring fluorescence spectral characteristics and/or fluorescence lifetime characteristics may be carried out.

### Short Description of the Figures

Hereinafter, specific embodiments are described referring to the drawings, wherein:
- Figure 1: a schematic drawing showing conjugate labels for marking a target structure and their components;
- Figure 2: is a schematic drawing showing a marker structure;
- Figure 3: is a schematic drawing showing a further marker structure;
- Figure 4: is a schematic drawing showing a further marker structure;
- Figure 5: is a schematic drawing showing various combinations of marker structures with affinity reagents and target structures;
- Figure 6: is a schematic drawing showing marker structures with preferred arrangements of labels; and
- Figure 7: is a schematic drawing showing marker structures with different optical characteristics.

### Detailed Description

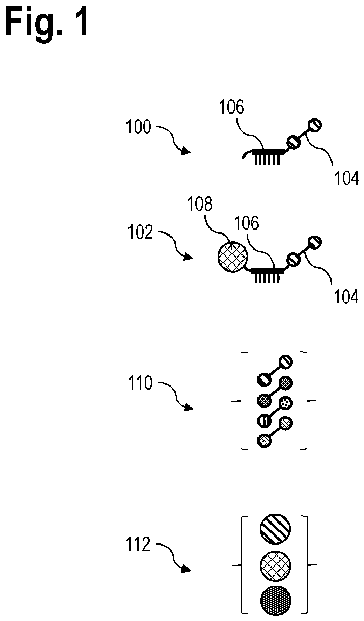

Figure 1 is a schematic drawing showing conjugate labels 100, 102 for marking a target structure and their components. Specifically, the label 100 may comprise a polyyne 104 with a distinct Raman spectroscopy characteristic and an oligonucleotide 106. Further, the label 102 may comprise the polyyne 104, the oligonucleotide 106 and a fluorescent dye 108 with a distinct spectral characteristic and/or a distinct fluorescent lifetime characteristic.

The conjugate labels 100, 102 may be generated based on a collection of polyyne 110 with different distinct Raman spectroscopy characteristics and based on a collection of fluorescent dyes 112 with different fluorescent characteristics, such as their fluorescent emission wavelengths and/or their fluorescent lifetime characteristics. By choosing different combinations of polyyne 110 and/or dyes 112 when generating labels, labels with a plurality of unique Raman and/or fluorescent characteristics may be generated.

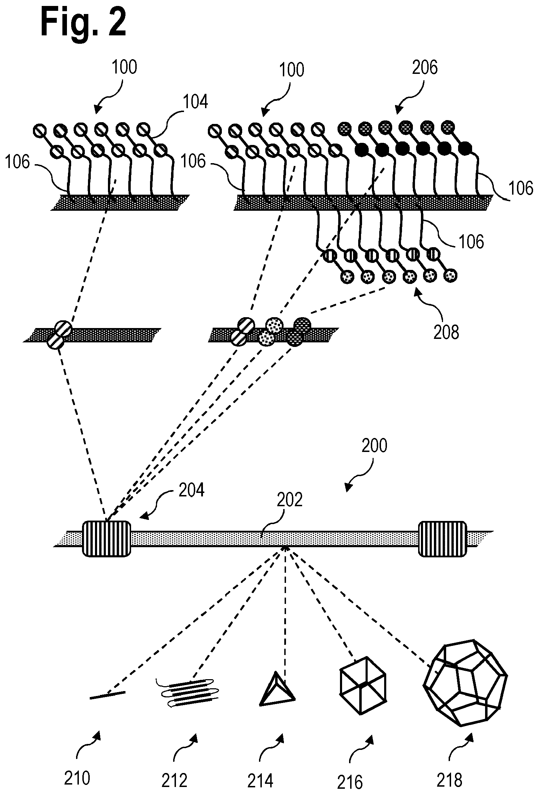

Figure 2 is a schematic drawing showing a marker structure 200. The marker structure 200 comprises a backbone 202 and plurality of labels 204 attached to the backbone 202.

The plurality of labels 204 may be limited to several labels 100 with polyynes with a single distinct Raman spectroscopy characteristic, or several groups of labels 100, 206, 208, where polyynes 110 of each group of labels 100, 206, 208 differ in their Raman spectroscopy characteristic.

The backbone 202 may be DNA nanotechnology-based, for example, based on DNA origami.

The backbone 202 may have different geometries 210, 212, 214, 216, 218. In particular, the backbone 202 being DNA-origami based, allows generating predetermined, stable two- and three-dimensional shapes. Further, this allows generating a plurality of attachment sites at predetermined positions along the backbone 202. The attachment sites are stretches of oligonucleotide, that are unique and allow the hybridisation of complementary oligonucleotides, for example to attach - directly or indirectly - the labels 100, 102, 206, 208 to the backbone 202 at these predetermined positions.

The geometry 210 is linear or rod-like. The geometry 212 is sheet-like, which may be a large linear DNA molecule or an assembly of multiple DNA molecules. Sheet-like geometries may increase the number of available attachment sites substantially. Further geometries are possible, for example, a tetrahedral geometry 214, cubic geometry 216, or a polyhedral geometry 218.

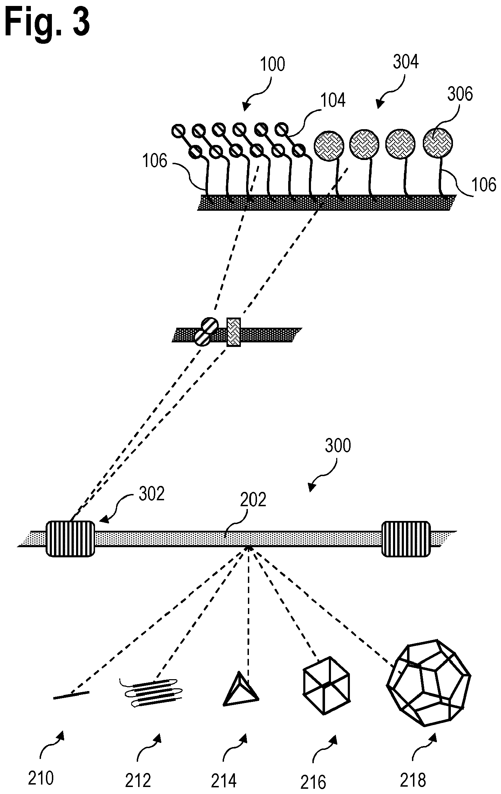

Figure 3 is a schematic drawing showing a marker structure 300. The marker structure 300 comprises the backbone 202, as described for Fig. 2, to which a plurality of labels 302 may be attached.

The plurality of labels 302 may comprise the label 100 with the polyyne 104 and a label 304 with a fluorophore 306. Alternatively, the marker structure 300 may comprise the label 102, with the polyyne 104 and the fluorophore 108.

In case of Figure 2 and 3, the oligonucleotide 106 of the labels 100, 102, 206, 208, 304 may have a sequence complementary to a part of the backbone 202, such that the labels 100, 102, 206, 208, 304 may attach to the backbone 202.

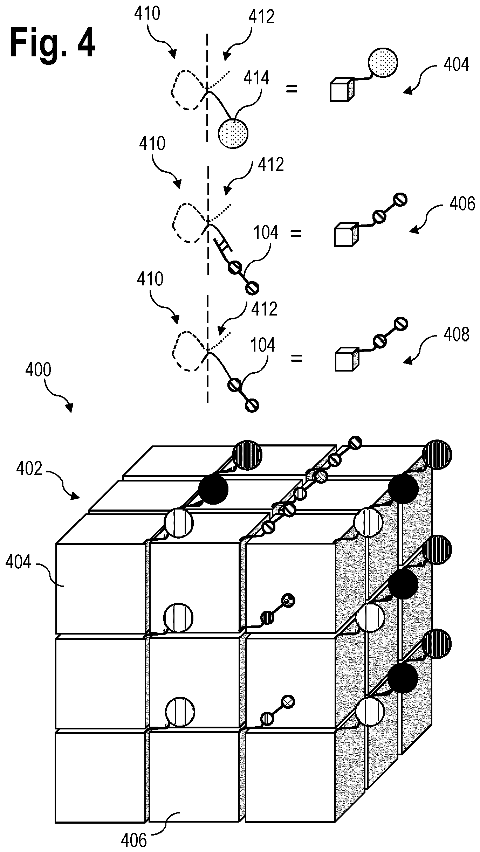

Figure 4 is a schematic drawing showing a marker structure 400. The marker structure 400 comprises a backbone 402 of DNA bricks. The individual DNA bricks may be labels 404, 406, 408. DNA bricks generally comprise an oligonucleotide head 410 and tail 412 portion that bind to the respective portions of other DNA bricks in order to generate the backbone 402. The labels 406, 408 may comprise polyynes 104 as described previously for Fig. 1. Additionally, the marker structure 400 may comprise optional labels 404, that comprise a fluorophore 414.

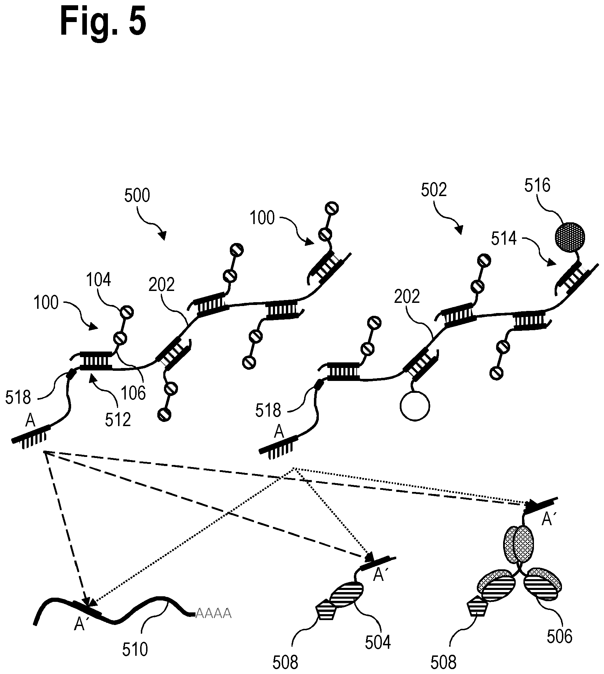

Figure 5 is a schematic drawing showing various combinations of marker structures 500, 502 with affinity reagents 504, 506 and target structures 508, 510. The marker structure 500 comprises labels 100 with polyynes 104 and oligonucleotides 106, and the backbone 202. The oligonucleotides 106 bind the labels 100 to the backbone 202 via complementary base pairing at binding sites 512. Marker structure 502 additionally comprises labels 514 comprising a fluorophore 516. The marker structure 500, 502 may additionally comprise labels 102 with polyynes 104 and fluorophores 108.

The marker structures 500, 502 may comprises labels with a plurality of distinct properties such as Raman spectroscopy characteristic and fluorescent lifetime and spectrum characteristics. This enables generating a large number of marker structures with distinct properties that may be distinguished from each other, in particular, by an optical readout, for example, by means of a microscope, a cytometer, or a fluorescence activated cell sorting (FACS) device.

The marker structures 500, 502, specifically the backbone 202, may comprise a binding site A for attaching the affinity reagents 504, 506 that comprise an oligonucleotide with a complementary binding site A'. The affinity reagents 504, 506 may in turn bind specifically to a particular analyte, such as the target structure 508. As an example, the affinity reagent 504 is an antibody fragment, the affinity reagent 506 is an antibody. In this case, the target structure 508 may be a protein.

Alternatively, the marker structures 500, 502 may directly bind to the target structure 510, in particular, in case the target structure 510 is an oligonucleotide such as an mRNA strand comprising the complementary binding site A'.

Optionally, the marker structures 500, 502, specifically the backbones 202, may comprise a cleavage site 518, such as a restriction site, which allows separating the labels 100, 514 from the marker structures 500, 502, or which allows separating the binding site A of the marker structures 500, 502 from at least some of the labels 100, 514. This enables iterative staining methods were marking target structures in samples with marker structures is followed by removal of at least the labels of the marker structures and subsequent marking of the samples with further marker structures. Such methods are disclosed in document PCT/EP2021/073819, for example.

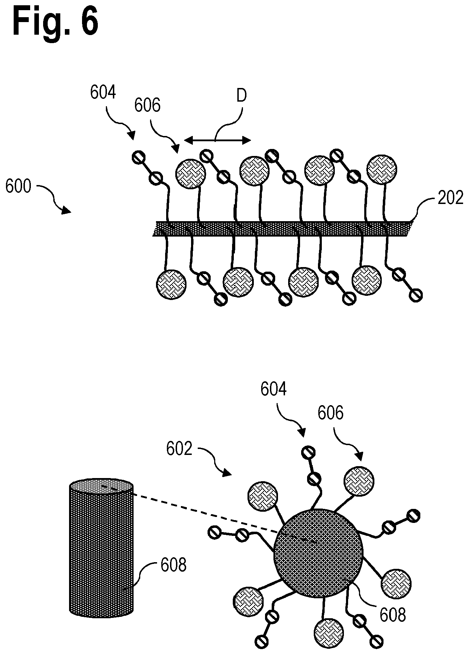

Figure 6 is a schematic drawing showing marker structures 600, 602 with particularly preferred arrangements of labels 604, 606. The marker structure 600 comprises polyyne-based labels 604 and fluorophore-based labels 606 attached to the backbone 202. The distance D between two adjacent or proximately arranged fluorophore-based labels 606 is preferably larger than 1.4 nm or a fluorophore-specific distance at which excitonic decoupling between adjacent fluorophores is ensured. Further, the polyyne-based labels 604 may be placed between adjacent labels 606 in an interleaved arrangement in order to excitonically decouple the fluorophore-based labels 606. Alternatively or additionally, as exemplified with the marker structure 602, the labels 604, 606 may be arranged around an essentially cylindrical backbone 608 in an interleaved helical fashion.

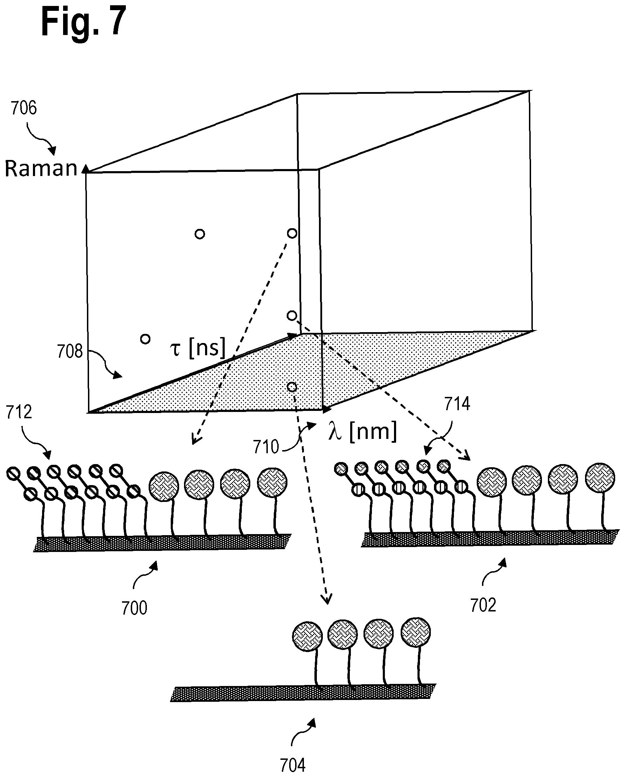

Figure 7 is a schematic drawing showing marker structures 700, 702, 704 with different optical characteristics in terms of their Raman spectroscopy characteristic 706, their fluorescent lifetime characteristic (τ) 708, and their fluorescent spectral characteristic (λ) 710. By combining different set of labels, each marker structure 700, 702, 704 may be distinguished based on the above optical characteristics. For example, marker structure 700 may be distinguished from marker structure 702 based on the different Raman spectroscopy characteristic of polyyne based label 712 and polyyne based label 714.

For example, from Hu et al., 2018 (Super-multiplexed optical imaging and barcoding with engineered polyynes, Nat Methods, 15(3): 194-200) engineered polyynes with distinct Raman characteristics are known. Thus, with fluorophores having 15 distinguishable spectral characteristics and 4 distinguishable fluorescent lifetimes and polyynes with 15 distinguishable Raman spectroscopy characteristics, a total of 1200 distinguishable marker structures may be generated.

This enables marking and identifying a plurality target structures in a sample, for example, a biological sample such as a histological section. To this end, the marker structure may be introduced into the sample and a target structure in the sample may be marked. This enables identifying the presence of the target structure in the sample. For example, a cytometer may be used to detect the presence of the target structure by measuring the Raman spectroscopy characteristic, the fluorescent spectral characteristic and/or the fluorescence lifetime characteristic of the marked sample. Based on this, the sample may be sorted by means of a cell sorter.

The marked sample may alternatively or additionally be imaged by means of a microscope. This enables locating a particularly marked target structure within the sample. By providing a plurality of distinguishable marker structures, a plurality of target structures may be simultaneously distinguished and identified.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

| | |
|---|---|
| 100, 102, 206, 208, 304, 404, 406, 408, 514, 604, 606, 712, 714 | Label |
| 104,110 | Polyyne |
| 106 108, 112, 306, | Oligonucleotide |
| 414, 516 200, 300, 400, 500, 502, 600, 602, 700, 702, | Fluorophore |
| 704 | Marker structure |
| 202, 402, 608 | Backbone |
| 204, 302 210, 212, 214, | Plurality of labels |
| 216, 218 | Backbone geometries |
| 410 | DNA brick head |
| 412 | DNA brick tail |
| 504, 506 | Affinity reagent |
| 508, 510 | Target structure |
| 512 | Binding site |
| 518 | Cleavage site |
| 706 | Raman spectroscopy characteristic |
| 708 | Fluorescent lifetime characteristic |
| 710 | Fluorescent spectral characteristic |

## Claims

1. A label (100, 102, 206, 208, 404, 406, 408, 514, 604, 606, 712, 714) for marking a target structure (508, 510), comprising at least one polyyne (104, 110) and at least one oligonucleotide (106), wherein the at least one polyyne (104, 110) is configured to be detectable via a distinct Raman spectroscopy characteristic.

2. The label according to claim 1, wherein the distinct Raman spectroscopy characteristic is a stimulated Raman scattering (SRS) characteristic or a coherent anti-Stokes Raman scattering (CARS) characteristic.

3. The label according to claim 1 or 2, further comprising at least one first fluorophore (108, 112, 414, 516) which is configured to be detectable via at least a distinct spectral characteristic.

4. The label according to one of the preceding claims, further comprising at least one second fluorophore (108, 112, 414, 516) which is configured to be detectable via at least a distinct fluorescence lifetime characteristic.

5. The label according to one of the preceding claims, comprising at least one conjugate which is formed from the at least one polyyne (104, 110) and the at least one oligonucleotide (106).

6. The label according to claim 5, wherein the at least one conjugate further includes the at least one first fluorophore (108, 112, 414, 516).

7. The label according to claim 5 or 6, wherein the at least one conjugate further includes the at least one second fluorophore (108, 112, 414, 516).

8. The label according to one of the preceding claims, wherein the label is configured to be attached to an affinity reagent (504, 506) binding to the target structure (508, 510).

9. A marker structure (200, 300, 400, 500, 502, 600, 602, 700, 702, 704) comprising a plurality of labels according to one of the preceding claims.

10. The marker structure according to claim 9, wherein the labels of the plurality of labels differ from each other at least in part with respect to the distinct Raman spectroscopy characteristics of their respective polyynes (104, 110), and/or the spectral characteristics of their first fluorophores, and/or the fluorescence lifetime characteristics of their second fluorophores.

11. The marker structure according to claim 9 or 10, comprising a backbone (202, 402, 608) on which the labels are attached.

12. The marker structure according to claim 11, wherein the backbone (202, 402, 608) is formed from a DNA nanotechnology-based structure or wherein the backbone is formed from a gel-like structure.

13. The marker structure according to claim 11 or 12, wherein the first fluorophores and/or the second fluorophores are excitonically decoupled by an interleaved arrangement with the polyynes along or around the backbone (202, 402, 608).

14. A method for examining a target structure, comprising the following steps:
marking the target structure (508, 510) with the marker structure according to one of the claims 9 to 13,
measuring Raman spectroscopy characteristic of the marked target structure, and
measuring fluorescence of the marked target structure.

15. The method according to claim 14, wherein measuring fluorescence of the marked target structure comprises detecting spectral characteristics of the marker structure and/or detecting fluorescence lifetime characteristics of the marker structure.

16. The method according to claim 14 or 15, wherein at least one of the Raman spectroscopy characteristic and the fluorescence are measured by means of a microscope.

17. The method according to claim 14 or 15, wherein at least one of the Raman spectroscopy characteristic and the fluorescence are measured by means of a cytometer.

18. The method according to claim 17, wherein the method further comprises the step of sorting the marked target structure based on the measured Raman spectroscopy characteristic and/or the measured fluorescence.
